Europäisches Patentamt

European Patent Office

Office européen des brevets

(19)

(11) Publication number: **0 234 863**

**A2**

(12) **EUROPEAN PATENT APPLICATION**

(21) Application number: **87301420.3**

(22) Date of filing: **19.02.87**

(51) Int. Cl.³: **C 12 N 1/06**
**C 12 P 1/00**

(30) Priority: **22.02.86 GB 8604440**

(43) Date of publication of application:
**02.09.87 Bulletin 87/36**

(84) Designated Contracting States:
**AT BE CH DE ES FR GB GR IT LI LU NL SE**

(71) Applicant: **Bovril Limited**
**Clayponds Lane**
**Brentford, Middlesex TW8 9BD(GB)**

(72) Inventor: **Hobson, John Charles**
**27 Nene Close Stretton**
**Burton-on-Trent Staffordshire DE13 0YA(GB)**

(72) Inventor: **Seidel, Hans**
**4 Lincoln Road Stapenhill**
**Burton-on-Trent Staffordshire DE15 9HP(GB)**

(74) Representative: **Lockwood, Barbara Ann et al,**
**Beecham Pharmaceuticals Biosciences Research Centre**
**Great Burgh Yew Tree Bottom Road**
**Epsom Surrey KT18 5XQ(GB)**

(54) **Process for rupturing cells.**

(57) A process for rupturing cells by thermal shock is disclosed in which a slurry or aqueous cells is treated with a fluid at a higher temperature and pressure than the slurry, sufficient to cause structural damage to the cells. The resultant slurry/fluid mixture is maintained at an elevated temperature for sufficient time to allow release of the intracellular material prior to cooling. Subsequently co-enzymes, co-factors and other intracellular products may be obtained from the cooled mixture. The process is readily applied to yeast cells, without preventing their subsequent use for the production of yeast extract.

EP 0 234 863 A2

C259

**TITLE MODIFIED**
see front page          PROCESS

The present invention relates to a process for treating cells, and in particular a process for obtaining intra-cellular material, such as co-enzymes, from ruptured cells.

It is known to obtain co-enzymes from yeast by batchwise chemical and/or physical methods. For example Japanese Patent No. 6140-947 (Sekisui Chemi Ind KK) discloses the preparation of a particular co-enzyme Q by acid treatment of yeast at elevated temperature. Such treatment destroys the food value of yeast as yeast extract and thus creates a waste product with little commercial value.

Furthermore, the treatment is generally only possible on a small scale with resulting minute quantities of co-enzymes.

A continuous process for treating cells, including yeast, has now been found whereby relatively large amounts of cells can be processed to separate intra-cellular material such as co-enzymes. When applied to yeast the food value is unaffected. The yeast cells and yeast cell debris which are by-products of the process can be utilised for the production of edible yeast extract, thus making the process economically viable on a large scale.

According to the present invention there is provided a process for rupturing cells by thermal shock which comprises:

(i)     generating a flow of a slurry of aqueous cells in a vessel;

(ii)    supplying to the flow of slurry a fluid at a higher temperature and pressure at the point of application than the slurry by an amount sufficient to cause structural damage to the cells and such that the temperature of the resulting slurry/fluid mixture is greater than 30°C;

(iii)   maintaining the slurry/fluid mixture at a temperature above 30°C for a time sufficient to allow release of intracellular material; and

(iv)    cooling it if necessary, to a temperature of below 40°C.

In a further aspect of the process of the invention, the cooled mixture obtained by step (iv) above is separated into cell material and cell free extract by conventional separation methods, and the extract is fractionated to obtain co-enzymes, co-factors and other intra-cellular products.

Preferably the cells to which the process of the invention is applied are microbial cells or fungal cells. However the process may also be applied to cellular matter such as biomass from an aerobic digester. Suitable microbial cells may include microorganisms of the genus Acetobacter, Aspergillus and Streptomyces. Suitable fungal cells, which terminology includes herein fungal hyphae, include yeasts such as Sacchaormycopsis and moulds such as Pencillium.

The cells may be alive or newly dead. Thus it will be apparant to those skilled in the art that the process of the invention may be applied to spent fermentation solids from the brewing industry, in the case of such yeasts without affecting their subsequent use as a yeast extract foodstuff. Alternatively the process can be applied to spent solids from the vinegar industry and from the production of β-lactam antibiotics obtained by fermentation processes.

Preferably the cell slurry has a total solids content of from about 2 to 20% by weight. The initial temperature of the slurry is usually not important, but is normally below 20°C.

The slurry may be fed into a vessel in the form of a conduit and the heated fluid supplied to the slurry flow via an inlet to the conduit so that the fluid flow is transverse to the slurry flow. This enables the fluid to mix fully with the slurry flow and rapidly to reach the required operating temperature.

Preferably, the slurry is fed through a gas entrapment nozzle which is located adjacent the inlet to the conduit. This enables the fluid to be 'sucked' into a stream of slurry droplets, which has been found to increase the efficiency of the process. The fluid is most preferably fed into the slurry stream at right angles to the direction of flow of the stream.

The slurry is preferably pumped through the conduit at an adjustable rate, which may be varied according to the temperature and pressure of the heated fluid.

In accordance with a preferred aspect of the invention there is provided a process for rupturing cells by thermal shock, which comprises,

- 4 -

(i)    generating a flow of aqueous slurry in a vessel,

(ii)    subjecting the flow to treatment with heated fluid supplied to the vessel at a pressure of from 0 to 600 K pascals and temperature of from 100 to 500$^{\circ}$C,

(iii)    adjusting the flow of heated fluid and/or yeast slurry so that the resulting slurry mixture is brought to a temperature of from 30$^{\circ}$C to 90$^{\circ}$C,

(iv)    maintaining the slurry mixture at a temperature of from 30$^{\circ}$C to 90$^{\circ}$C for between 30 secs and 10 mins, and

(v)    cooling the mixture to below 40$^{0}$C.

It has been found that no further benefit is achieved when stage (iv) is carried out for longer than 10 minutes, and moreover it is not desirable to keep the temperature above 40$^{\circ}$C for a more extended period as this can cause degradation of the cell products.

Preferably the cells are yeast cells comprising brewery and/or bakers yeast.

The preferred fluid is steam which, due to its latent heat capacity, can provide the necessary heat energy to cause thermal shock of the yeast cells.  Furthermore, it is relatively safe and easy to control the temperature and pressure of steam.

Alternative fluids which may be used are inert gases (such as nitrogen) or hot water.

The preferred pressure range for the fluid is from 10 to 600 K pascals, and for steam a range of from about 50 to 60 K pascals is particularly suitable. The preferred temperature range for the fluid is from 100 to 250°C, and for steam a range of from 100 to 160°C may be employed.

The resulting initial mixture of slurry and heated fluid is preferably brought to a temperature range of about 50 to 75°C in the mixing region, and is subsequently maintained at this temperature range for preferably from 1 minute to 4 minutes, more preferably for about 2 to 3 minutes.

The required temperature of the initial mixture may be achieved by adjusting the flow rates of the heated fluid and slurry, together with the temperature and pressure of the heated fluid. This may be done in a conventional manner by, for example employing a temperature probe located in the mixed stream, which is coupled to an electronic temperature controller. The controller may be connected through an electropneumatic converter to an air-operated modulating steam control valve. Alternatively, a manually operated steam control valve can be employed, the operator first reading the temperature of the initial mixture and subsequently increasing the steam flow to increase the temperature, or reducing the flow to decrease the temperature.

The initial mixture is preferably fed into a holding vessel in which the mixture is stirred or agitated for the required ''residence'' time, and the holding vessel may be insulated and/or heated to provide the necessary temperature range.

In a preferred embodiment of the invention, the holding
vessel consists of a chamber having an overflow duct,
the height of the duct above the base of the chamber
dictating the residence time of mixture in the chamber.

After leaving the holding vessel, the mixture is
cooled, if necessary, to below 40°C, preferably by
passing it through a heat exchanger. Suitably the
mixture is cooled to below 20°C in less than 2 minutes,
more preferably in about 30 seconds or less, and
conventional heat exchangers may be used for this
purpose. For example, the mixture may be pumped
through a column surrounded by a jacket of cooling
medium, such as cold water.

The cooled slurry mixture is then separated into two
main components, namely;

(i)     intact cells and cell debris, and
(ii)    cell free extract,

by centrifugation, pressing, ultra filtration or a
mixture of these methods.

Preferably, centrifugation is employed followed by
ultrafiltration, and the cell free extract thereby
obtained will contain a complex mixture of co-enzymes,
co-factors and other low molecular weight
intra-cellular products. The extract can itself be
further fractionated to isolate particular co-enzymes
or co-factors of interest, examples of which are acetyl
Co A, A.T.P. (adenosine 5' triphosphate), N.A.D.
(Nicotinamide-adenine dinucleotide), and N.A.D.P.
(Nicotinamide -adenine dinucleotide phosphate). These
co-enzymes and co-factors are themselves valuable
materials, and the process of the invention thus provides a straightforward and efficient way of extracting these materials from cells on a large-scale, continuous basis.

A particular advantage of the process of the invention is that when applied to yeasts the treated yeast cells and cell debris may be made into yeast extract (a valuable foodstuff) in the same way as untreated yeast, so there are no wasteful by-products of the process. This is important economically, since large quantities of yeast slurry must be processed to yield relatively small amounts of valuable co-enzymes and co-factors.

The process of the invention may be modified by pre-treating the cell slurry with certain enzymes prior to the thermal shock. This has been found to increase the final yield of co-enzymes and co-factors.

Preferably the enzymes used in the pre-treatment should comprise single or mixed activities of the following types:

(i)     Activity towards 1-3 linkages in the glucan
(ii)    Cellulase activity
(iii)   Laminarinase activity
(iv)    Xylanase activity
(v)     Chitinase activity
(vi)    Proteinase activity
(vii)   Glucanase activity

Generally, the cell slurry is mixed with an enzyme preparation containing activities (i) to (vii) such as *Novozym 234, in an amount from 0.05 to 0.5% w/v and incubated at a temperature of from 20°C to 40°C for between 30 minutes and 24 hours. The treated slurry is then subjected to thermal shock as hereinbefore described.

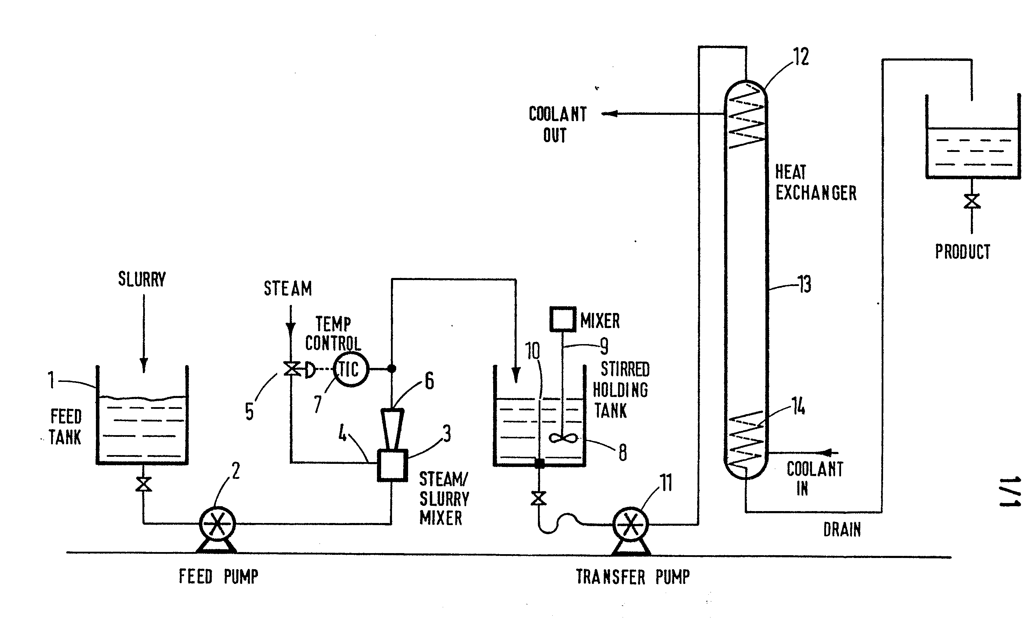

Examples of the invention are now described with reference to the accompanying drawing which is a flow diagram of apparatus for treating cell slurry by thermal shock using steam.

A yeast slurry was prepared by mixing 10 kg of fresh pressed brewery yeast with 10 kg of water, the mixture being agitated until a smooth slurry was formed. The percentage total solids content in the slurry was 12%.

The slurry was introduced into a feed tank 1 and pumped, using a feed pump 2, into the water input of a Baird and Tatlock plated metal filter pump 3, at a rate of 779 ml/minute.

Steam was pumped into the side arm, air inlet 4 of the filter pump 3, at a pressure of about 55 K pascals and temperature of about 113°C. The flow of steam was regulated manually using a BRV Sarco Spirax valve 5 so as to maintain the temperature of the slurry mixture leaving the outlet 6 of the filter pump 3 at 65°C, the temperature being monitored by a temperature probe 7, which was linked to the control valve 5. (The steam supply was trapped to remove condensed moisture and a safety valve prevented the pressure from rising above 55 K pascals.) The slurry mixture was passed into an insulated holding tank 8 and agitated with a mechanical stirrer 9 throughout the residence time of the slurry in the tank 8.

*Novozym 234 is a product of Novo Enzymes Products.

The tank 8 contained an overflow duct 10 down which
slurry passed to be pumped by a transfer pump 11 to the
top of a heat exchanger 12. The duct 10 was positioned
so that slurry would have an average residence time in
the tank of about three minutes before passing down the
duct. During this time the temperature was maintained
at about 62°C.

The heat exchanger 12 consisted of a cylindrical,
hollow jacket 13 of 1.5m length, containing a helical
coil of glass tubing 14, cold water at about 10°C being
pumped up the jacket 13 while slurry passed down the
tubing 14. The temperature of material obtained from
the bottom outlet of the tubing was 16°C.

The material was subsequently centrifuged to separate
solids from liquid, and the liquid then ultra
centrifuged to obtain an extract rich in co-enzymes,
co-factors and other intra-cellular material.

Example 2

The process was carried out in a similar manner to that
of Example 1, except that the yeast slurry was
pretreated with Novozym 234.

CLAIMS

1.    A process for rupturing cells by thermal shock which comprises:

(i)    generating a flow of a slurry of aqueous cells in a vessel;

(ii)    supplying to a flow of slurry a fluid at a higher temperature and pressure at the point of application than the slurry by an amount sufficient to cause structural damage to the cells and such that the temperature of the resulting slurry/fluid mixture is greater than 30°C,

(iii)    maintaining the slurry/fluid mixture at a temperature above 30°C for a time sufficient to allow release of intracellular material; and

(iv)    cooling, if necessary, to a temperature of below 40°C.

2.    A process as claimed in claim 1 wherein the cells are microbial or fungal cells or a cellular biomass.

3.    A process as claimed in claim 1 or claim 2 wherein the cells are yeast cells.

4.    A process as claimed in any preceding claim which comprises:

(i)    generating a flow of aqueous cell slurry in a vessel,

(ii)    subjecting the flow to treatment with heated fluid supplied to the vessel at a pressure of from 0 to 600 K pascals and a temperature of from 100 to 500°C,

(iii)    adjusting the flow of heated fluid and/or yeast slurry so that the resulting slurry mixture is brought to a temperature of from 30°C to 90°C,

(iv)    maintaining the slurry mixture at a temperature of from 30°C to 90°C for between 30 seconds and 10 minutes, and

(v)    cooling the mixture to below 40°C.

5.    A process as claimed in any preceeding claim in which the heated fluid is steam.

6.    A process as claimed in claim 5 in which the steam is supplied at a pressure of from 50 to 60 k pascals and a temperature of from 100°C to 160°C.

7.    A process as claimed in claim 4 in which the temperature in steps (iii) and (iv) is from 50 to 75°C.

8.    A process as claimed in any preceding claim in which the slurry of aqueous cell has a total solids content of from 2 to 20% by weight prior to thermal shock treatment.

9.    A process as claimed in any preceding claim in which the cell slurry is fed into a conduit and the heated fluid is supplied to the slurry flow via an inlet to the conduit.

10. A process as claimed in any preceding claim comprising the additional step of separating the cooled mixture obtained by cooling to a temperature below 40°C into cell material and cell free extract.

11. A process as claimed in claim 10, in which the cell free extract is fractionated to obtain co-enzymes, co-factors and other intra-cellular products.

12. Co-enzymes, co-factors and other intra-cellular products obtained by the process of claim 11.

SLURRY

STEAM

COOLANT OUT

12

HEAT EXCHANGER

1

FEED TANK

2

FEED PUMP

5

7

TEMP CONTROL

TIC

4

3

STEAM/ SLURRY MIXER

6

10

MIXER

9

STIRRED HOLDING TANK

8

11

TRANSFER PUMP

13

14

COOLANT IN

DRAIN

PRODUCT

1/1

0234863